(19) Europäisches Patentamt
European Patent Office
Office européen des brevets

(11) Veröffentlichungsnummer: **0 160 620**
**B1**

# (12) EUROPÄISCHE PATENTSCHRIFT

(45) Veröffentlichungstag der Patentschrift: **13.09.89**

(51) Int. Cl.⁴: **C 07 D 249/08**, C 10 M 133/44 // C10N30:14

(21) Anmeldenummer: **85810138.9**

(22) Anmeldetag: **29.03.85**

(54) **Neue Metalldesaktivatoren.**

(30) Priorität: **04.04.84 GB 8408617**

(43) Veröffentlichungstag der Anmeldung: **06.11.85 Patentblatt 85/45**

(45) Bekanntmachung des Hinweises auf die Patenterteilung: **13.09.89 Patentblatt 89/37**

(84) Benannte Vertragsstaaten: **BE DE FR IT NL**

(56) Entgegenhaltungen:
**EP-A-0 106 243**

**CHEMICAL ABSTRACTS, Band 94, Nr. 23, 8. Juni 1981, Seite 652, Zusammenfassung Nr. 192228r, Columbus, Ohio, US; T. BANY et al.: "Cyanoethylation and aminomethylation of 1,2,4-triazole"**
**CHEMICAL ABSTRACTS, Band 94, Nr. 13, 30. März 1981, Seite 734, Zusammenfassung Nr. 103249h, Columbus, Ohio, US; T. BANY et al.: "Cyanoethylation and aminomethylation of 1,2,4-triazole"**

(73) Patentinhaber: **CIBA- GEIGY AG, Klybeckstrasse 141, CH- 4002 Basel (CH)**

(72) Erfinder: **Phillips, Emyr, Dr., 12 Pinewood Court Broad Road, Sale Cheshire M33 2ES (GB)**
Erfinder: **Holt, Brian, Dr., 70 Moss Park Road, Stretford Manchester M32 9HQ (GB)**

**EP 0 160 620 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Zusammensetzungen, enthaltend Triazole, neue Triazole und deren Herstellung und Verwendung als Metalldesaktivatoren in funktionellen Flüssigkeiten.

Aus Chemical Abstracts, Band 94, 103 249 h und 192 228r ist die Aminomethylierung von 1,2,4-Triazol bekannt und es werden 1-Morpholinomethyl-, 1-Piperidinomethyl, 1-Diphenylaminomethyl- und 1-N-Phenyl-N-Benzylaminomethyl-triazol beschrieben. Aus der EP-A-0 106 243 sind weitere Verbindungen der Azolyl-methylamine, deren Herstellung und deren Verwendung in mikrobiziden Mitteln bekannt.

Es wurden neue Zusammensetzungen und weitere neue Triazole gefunden.

Gegenstand der vorliegenden Verbindung sind neue Zusammensetzungen enthaltend eine funktionelle Flüssigkeit und, als Metalldesaktivator, einen metalldesaktivierenden Anteil einer Verbindung der Formel I

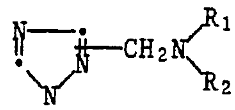

I

worin

$R_1$ und $R_2$, gleich oder verschieden, $C_1$-$C_{20}$-Alkyl, $C_3$-$C_{20}$-Alkenyl, $C_5$-$C_{12}$-Cycloalkyl, $C_7$-$C_{13}$-Aralkyl, $C_6$- oder $C_{10}$-Aryl bedeuten, oder zusammen mit dem Stickstoffatom, an das sie gebunden sind, einen 5 - 7 gliedrigen heterozyklischen Rest bilden,

oder worin

$R_1$ und $R_2$ einen Rest der Formel II

$R_3X[(Alkylen)O]_n$-(Alkylen)- II

bedeuten, worin

X O, S oder N,

$R_3$ Wasserstoff oder $C_1$-$C_{20}$-Alkyl und Alkylen einen $C_1$-$C_{12}$-Alkylen-Rest, bevorzugt einen $C_1$-$C_6$-Alkylen-Rest, insbesondere bevorzugt einen $C_2$-$C_3$-Alkylen-Rest und

n Null oder eine ganze Zahl von 1 bis 6, bevorzugt Null und 1 - 4 bedeuten,

oder

$R_1$ hat die vorstehend beschriebene Bedeutung und

$R_2$ bedeutet einen Rest der Formel III

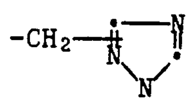

III

oder

$R_2$ bedeutet einen Rest der Formel III wie vorstehend definiert und

$R_1$ bedeutet einen Rest der Formel IV

$-[Alkylen]_n-N(R_4)-A-[N(R_4)_2]_m$ IV

worin

m Null oder 1 ist und

A im Fall von m = Null einen Rest der Formel III und im Fall von m = 1 Alkylen oder $C_6$- oder $C_{10}$-Arylen bedeutet und Alkylen und

n die vorstehend definierten Bedeutungen haben und

$R_4$ einen Rest der Formel III, wie vorstehend beschrieben, bedeutet.

Ein weiterer Gegenstand der vorliegenden Erfindung sind neue N-substituierte Verbindungen der Formel I

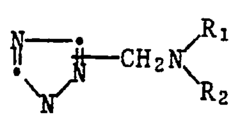

I

worin

$R_1$ und $R_2$, gleich oder verschieden, $C_1$-$C_{20}$-Alkyl, $C_3$-$C_{20}$-Alkenyl, $C_5$-$C_{12}$-Cycloalkyl, $C_7$-$C_{13}$-Aralkyl, $C_6$- oder $C_{10}$-Aryl bedeuten, oder zusammen mit dem Stickstoffatom, an das sie gebunden sind, einen 5 - 7 gliedrigen heterozyklischen Rest bilden,

oder worin

$R_1$ und $R_2$ einen Rest der Formel II

$R_3X[(Alkylen)O]_n(Alkylen)-$ II

bedeuten, worin

X O, S oder N,

$R_3$ Wasserstoff oder $C_1$-$C_{20}$-Alkyl und Alkylen einen $C_1$-$C_{12}$-Alkylen-Rest, bevorzugt einen $C_1$-$C_6$-Alkylen-Rest, insbesondere bevorzugt einen $C_2$-$C_3$-Alkylen-Rest und

n Null oder eine ganze Zahl von 1 bis 6, bevorzugt Null und 1 - 4 bedeuten,

oder

$R_1$ hat die vorstehend beschriebene Bedeutung und

$R_2$ bedeutet einen Rest der Formel III

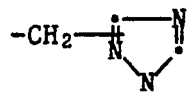

III

oder

$R_2$ bedeutet einen Rest der Formel III wie vorstehend definiert und

$R_1$ bedeutet einen Rest der Formel IV

$-[Alkylen]_n-N(R_4)-A-[N(R_4)_2]_m$ IV

worin

m Null oder 1 ist und

A im Fall von m = Null einen Rest der Formel III und im Fall von m = 1 Alkylen oder $C_6$- oder $C_{10}$-Arylen bedeutet und Alkylen und

n die vorstehend definierten Bedeutungen haben und

$R_4$ einen Rest der Formel III, wie vorstehend beschrieben,

bedeutet, mit der Massgabe, dass die Verbindungen 1-N-Phenyl-N-Benzylaminomethyltriazol, 1-Di-phenylaminomethyl-triazol, 1-(-4-morpholinomethyl)-triazol und 1-(-4-piperidinomethyl)-triazol ausgenommen sind.

Sind $R_1$ und/oder $R_2$ und/oder $R_3$ $C_1$-$C_{20}$-Alkyl, kann es sich um geradkettige oder verzweigte Substituenten handeln wie z. B. Methyl, Ethyl, n-Propyl, i-Propyl, n-Butyl, sec-Butyl, n-Pentyl, n-Hexyl, n-Heptyl, n-Octyl, 2-Ethylhexyl, n-Nonyl, n-Decyl, n-Dodecyl, n-Tetradecyl, n-Hexadecyl, n-Octadecyl oder n-Eicosyl.

Sind $R_1$ und/oder $R_2$ $C_3$-$C_{20}$-Alkenyl, so handelt es sich um geradkettige oder verzweigte Substituenten wie z. B. Prop-2-enyl, But-2-enyl, 2-Methylprop-2-enyl, Pent-2-enyl, Hexa-2,4-dienyl, Dec-10-enyl oder Eicos-2-enyl.

Sind $R_1$ und/oder $R_2$ $C_5$-$C_{12}$-Cycloalkyl so handelt es sich z. B. um Cyclopentyl, Cyclohexyl, Cyclooctyl, Cyclodecyl, Adamantyl oder Cyclododecyl.

Sind $R_1$ und/oder $R_2$ $C_7$-$C_{13}$-Aralkyl so handelt es sich z. B. um Benzyl, 2-Phenylethyl, Benzhydryl oder Naphthylmethyl.

Sind $R_1$ und/oder $R_2$ $C_6$- oder $C_{10}$-Aryl so handelt es um Phenyl oder Naphthyl.

Sind $R_1$ und $R_2$ zusammen mit dem Stickstoffatom an das sie gebunden sind, ein Heterozyklus, so handelt es sich z. B. um einen Morpholin-, Pyrrolidin-, Piperidin- oder Perhydroazepin-Rest.

Bei $C_1$-$C_{12}$-Alkylen der Reste der Formel II und IV handelt es sich z. B. um Methylen, Ethylen, 1,2-Propylen oder 1,3-Propylen, 1,4-Butylen, 1,6-Hexylen, 1,8-Octylen, 1,10-Decylen und 1,12-Dodecylen.

$C_6$- oder $C_{10}$-Arylen im Rest der Formel IV bedeutet Phenylen resp. Naphthylen.

Beispiele von Verbindungen der Formel I sind:

1-(oder 4-)Dimethylaminomethyl-triazol
1-(oder 4-)Diethylaminomethyl-triazol
1-(oder 4-)Diisopropylaminomethyl-triazol
1-(oder 4-)Di-n-butylaminomethyl-triazol
1-(oder 4-)Di-n-hexylaminomethyl-triazol
1-(oder 4-)Di-n-octylaminomethyl-triazol
1-(oder 4-)Di-n-decylaminomethyl-triazol
1-(oder 4-)Di-n-dodecylaminomethyl-triazol
1-(oder 4-)Di-n-octadecylaminomethyl-triazol
1-(oder 4-)Di-n-eicosylaminomethyl-triazol
1-(oder 4-)Di-prop-2-enylaminomethyl-triazol
1-(oder 4-)Di-but-2-enylaminomethyl-triazol
1-(oder 4-)Di-eicos-2-enylaminomethyl-triazol
1-(oder 4-)Di-benzylaminomethyl-triazol
1-(oder 4-)Di-phenylaminomethyl-triazol
1-(oder 4-)(-4-morpholinomethyl)-triazol
1-(oder 4-)(-4-pyrrolidinomethyl)-triazol
1-(oder 4-)(-4-piperidinomethyl)-triazol

1-(oder 4-)(-4-perhydroazepinomethyl)-triazol
1-(oder 4-)[(2,2'-dihydroxyethyl)aminomethyl]-triazol
1-(oder 4-)(Dibutoxypropyl-aminomethyl)-triazol
1-(oder 4-)(Dibutylthiopropyl-aminomethyl)-triazol
1-(oder 4-)(Dibutylaminopropyl-aminomethyl)-triazol
N,N-bis-(1- oder 4-triazolylmethyl)-laurylamin
N,N-bis-(1- oder 4-triazolylmethyl)-oleylamin
N,N-bis-(1- oder 4-triazolylmethyl)-ethanolamin
N,N,N',N'-tetra(1- oder 4-triazolylmethyl)-ethylen-diamin.

Die vorliegende Erfindung betrifft im weiteren ein Verfahren zur Herstellung der Verbindungen der Formel I, dadurch gekennzeichnet, dass man eine Verbindung der Formel V

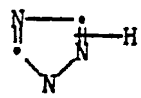

V

mit Formaldehyd und einem Amin der Formel VI,

$HNR_1R_2$ VI

worin $R_1$ und $R_2$ die vorstehend beschriebene Bedeutung haben, umsetzt.

Die Formaldehyd-Komponente wird geeigneterweise als Formalin oder Paraformaldehyd eingesetzt.

Die relativen Verhältnisse der Reaktionskomponenten der Formel V und VI und von Formaldehyd, die eingesetzt werden, hängen davon ab ob das Amin der Formel VI ein primäres oder sekundäres Amin oder ein Di-, Tri- oder Tetraamin ist.

Ist das Amin der Formel VI ein primäres Amin, beträgt das Molverhältnis an eingesetzter Verbindung der Formel V : Formaldehyd : Amin der Formel VI vorzugsweise 2 : 2 : 1 oder 1 : 1 : 1.

Ist das Amin der Formel VI ein sekundäres Amin, beträgt das Molverhältnis vorzugsweise 1 : 1 : 1, und wenn das Amin der Formel VI ein Diamin ist, beträgt das Molverhältnis vorzugsweise 4 : 4 : 1.

Die Umsetzung der Reaktionskomponenten miteinander nach dem erfindungsgemässen Verfahren erfolgt geeigneterweise durch Erhitzen bei höherer Temperatur, vorzugsweise in einem Temperaturbereich von 50 - 120°C, eventuell in Gegenwart eines inerten Lösungsmittels wie Wasser, $C_1$-$C_4$-Alkanol einem Petroläther wie z. B. Hexan, Isooctan oder Cyclohexan oder einem aromatischen Lösungsmittel wie z. B. Benzol, Toluol oder Xylol.

Andererseits kann die Verbindung der Formel V in einer ersten Stufe mit Formaldehyd zur N-Methylolverbindung umgesetzt werden, welche anschliessend mit dem Amin der Formel VI umgesetzt wird.

Eine weitere Möglichkeit besteht in der Umsetzung einer Verbindung der Formel V mit der entsprechenden Aminmethylolverbindung.

Die neuen Verbindungen der Formel I weisen wertvolle Eigenschaften als Metalldesaktivatoren in funktionellen Flüssigkeiten und als Schaumhemmer in Druckfarben für den Einsatz zur Herstellung von texturierten verschäumten Harzmaterialien (wie in der US-Patentschrift 4 407 882 beschrieben) auf.

Die vorliegende Erfindung betrifft im weiteren eine Zusammensetzung enthaltend eine funktionelle Flüssigkeit und als Metalldesaktivator, einen metalldesaktivierenden Anteil, vorzugsweise 0.001 Gew.-% bis 5 Gew.-% bezogen auf das gesamte Stoffgemisch, einer Verbindung der Formel I.

Beispiele von funktionellen Flüssigkeiten, die als Substrate für die erfindungsgemässen Zusammensetzungen dienen, sind Schmiermittel auf Mineralöl-, Poly-alpha-Olefin- oder synthetischer Carbonsäureester-Basis, Hydraulikflüssigkeiten auf der Basis von Mineralöl, Phosphatester, wässrigen Polyglycol/Polyglycolether-Mischungen Glycolsystemen, Öl-in-Wasser- oder anderen Wasser-in-Öl-Emulsionen; Flüssigkeiten zur Metallbearbeitung auf der Basis von Mineralöl oder wässrigen Systemen,

Kühlzusammensetzungen für Motoren auf der Basis von Wasser, wässrigem Glycol oder Ethylen- oder Propylenglycol/Methanol sowie Transformatoren- und Schalteröle.

Beispiele für synthetische Schmiermittel umfassen Schmiermittel auf der Basis eines Diesters einer zweiwertigen Säure und eines einwertigen Alkohols wie z. B. Dioctylsebacat oder Dinonyladipat, eines Triesters von Trimethylolpropan mit einer einwertigen Säure oder Mischungen solcher Säuren wie z. B. Trimethylolpropantripelergonat, Trimethylolpropantricaprylat oder Mischungen davon, eines Tetraesters von Pentaerythrit und einer einwertigen Säure oder Mischungen solcher Säuren wie z. B. Pentaerythrit-tetracaprylat, oder auf der Basis komplexer Ester aus ein- oder zweiwertigen Säuren mit mehrwertigen Alkoholen wie z. B. komplexe Ester aus Trimethylolpropan mit Caprinsäure und Sebacinsäure oder von Mischungen davon.

Weitere synthetische Schmiermittel sind die dem Fachmann geläufigen und z. B. im "Schmiermittel-Taschenbuch" (Huethig Verlag, Heidelberg 1974) beschrieben. Besonders geeignet sind, neben den bevorzugten Mineralölen, z. B. Phosphate, Glycole, Polyglycole, Polyalkylenglycole und Poly-alpha-Olefine.

Von besonderem Interesse sind funktionelle Flüssigkeiten auf Wasserbasis wie z. B. Frostschutzmittel, Hydraulikflüssigkeiten und Flüssigkeiten zur Metallbearbeitung. Bevorzugt, zum Einsatz in diesen Flüssigkeiten

auf Wasserbasis, sind die Verbindungen der Formel I, worin $R_1$ und/oder $R_2$ ein hydrophiler Rest der Formel II sind.

Andererseits sind beim Einsatz in funktionellen Flüssigkeiten auf Öl-Basis Verbindungen der Formel I bevorzugt, worin $R_1$ und/oder $R_2$ eine lipophile Gruppe ist.

Die Zusammensetzungen können zusätzlich noch andere Additive enthalten, die zugegeben werden, um die Grundeigenschaften der jeweiligen funktionellen Flüssigkeiten noch weiter zu verbessern; dazu gehören: Antioxidantien, weitere Metalldesaktivatoren, Rostinhibitoren, Viskositätsindex-Verbesserer, Stockpunkterniedriger, Dispergiermittel, Detergentien sowie Antiverschleiss-Additive für Systeme auf Öl-Basis; ferner Antioxidantien, Korrosions- und Rostinhibitoren, weitere Metalldesaktivatoren, Hochdruck-Zusätze, Antiverschleiss-Additive, Komplexierungsmittel, Ausfällungsinhibitoren, Biocide, Puffersubstanzen und Schaumbrecher für Systeme auf Wasser Basis.

Beispiele von weiteren Additiven für Systeme auf Öl-Basis.

**1. Antioxidantien**

**1.1. Alkylierte Monophenole**

2,6-Di-tert.-butyl-4-methylphenol
2,6-Di-tert.-butylphenol
2-Tert.-butyl-4,6-dimethylphenol
2,6-Di-tert.-butyl-4-ethylphenol
2,6-Di-tert.-butyl-4-n-butylphenol
2,6-Di-tert.-butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.-butyl-4-methoxymethylphenol

**1.2. Alkylierte Hydrochinone**

2,6-Di-tert.-butyl-4-methoxyphenol
2,5-Di-tert.-butyl-hydrochinon
2,5-Di-tert.-amyl-hydrochinon
2,6-Diphenyl-4-octadecyloxyphenol

**1.3. Hydroxylierte Thiodiphenylether**

2,2'-Thio-bis-(6-tert.-butyl-4-methylphenol)
2,2'-Thio-bis-(4-octylphenol)
4,4'-Thio-bis-(6-tert.-butyl-3-methylphenol)
4,4'-Thio-bis-(6-tert.-butyl-2-methylphenol)

**1.4. Alkyliden-Bisphenole**

2,2'-Methylen-bis-(6-tert.-butyl-4-methylphenol)
2,2'-Methylen-bis-(6-tert.-butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.-butylphenol)
2,2'-Ethyliden-bis-(4,6-di-tert.-butylphenol)
2,2'-Ethyliden-bis-(6-tert.-butyl-4-isobutylphenol)
2,2'-Methylen-bis-[6-(α-methylbenzyl)-4-nonylphenol]
2,2'-Methylenbis-[6-(α,α-dimethylbenzyl)-4-nonylphenol]
4,4'-Methylen-bis-(2,6-di-tert.-butylphenol)
4,4'-Methylen-bis-(6-tert.-butyl-2-methylphenol)

1,1-Bis-(5-tert.-butyl-4-hydroxy-2-methylphenyl)-butan
2,6-Di-(3-tert.-butyl-5-methyl-2-hydroxybenzyl)-4-methylphenol
1,1,3-Tris-(5-tert.-butyl-4-hydroxy-2-methylphenyl)-butan
1,1-Bis-(5-tert.-butyl-4-hydroxy-2-methylphenyl)-3-n-dodecylmercapto-butan
Ethylenglycol-bis-[3,3-bis-(3'-tert.-butyl-4'-hydroxyphenyl)-butyrat]
Di-(3-tert.-butyl-4-hydroxy-5-methylphenyl)-dicyclopentadien
Di-[2-(3'-tert.-butyl-2'-hydroxy-5'-methyl-benzyl)-6-tert.-butyl-4-methyl-phenyl]-terephthalat.

**1.5 Benzylverbindungen**

1,3,5-Tri-(3,5-di-tert.-butyl-4-hydroxybenzyl)-2,4,6-trimethylbenzol
Di-(3,5-di-tert.-butyl-4-hydroxybenzyl)-sulfid
3,5-di-tert.-butyl-4-hydroxybenzyl-mercaptoessigsäure-isooctylester
Bis-(4-tert.-butyl-3-hydroxy-2,6-dimethylbenzyl)dithiol-terephthalat
1,3,5-Tris-(3,5-di-tert.-butyl-4-hydroxybenzyl)-isocyanurat
1,3,5-Tris-(4-tert.-butyl-3-hydroxy-2,6-dimethylbenzyl)-isocyanurat
3,5-Di-tert.-butyl-4-hydroxybenzyl-phosphonsäure-dioctadecylester
3.5-Di-tert.-butyl-4-hydroxybenzyl-phosphonsäure-monoethylester, Calcium-salz.

**1.6. Acylaminophenole**

4-Hydroxy-laurinsäureanilid
4-Hydroxy-stearinsäureanilid
2,4-Bis-octylmercapto-6-(3,5-di-tert.-butyl-4-hydroxyanilino)-s-triazin
N-(3,5-di-tert.-butyl-4-hydroxyphenyl)-carbaminsäureoctylester.

**1.7. Ester der β-(3,5-Di-tert.-butyl-4-hydroxyphenyl)-propionsäure** mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

**1.8. Ester der β-(5-tert.-butyl-4-hydroxy-3-methylphenyl)-propionsäure** mit ein- oder mehrwertigen Alkoholen, wie z. B. mit

| | |
|---|---|
| Methanol | Diethylenglycol |
| Octadecanol | Triethylenglycol |
| 1.6-Hexandiol | Pentaerythrit |
| Neopentylglycol | Tris-hydroxyethyl-isocyanurat |
| Thiodiethylenglycol | Di-hydroxyethyl-oxalsäurediamid |

**1.9. Amide der β-(3,5-Di-tert.-butyl-4-hydroxyphenyl)-propionsäure,** wie z. B.

N,N'-Di-(3,5-di-tert.-butyl-4-hydroxyphenylpropionyl)-hexamethylen-diamin
N,N'-Di-(3,5-di-tert.-butyl-4-hydroxyphenylpropionyl)-trimethylendiamin
N,N'-Di-(3,5-di-tert.-butyl-4-hydroxyphenylpropionyl)-hydrazin

**Beispiele für aminische Antioxidantien:**

N,N'-Di-isopropyl-p-phenylendiamin
N,N'-Di-sec.-butyl-p-phenylendiamin

N,N'-Bis(1,4-dimethyl-pentyl)-p-phenylendiamin
N,N'-Bis(1-ethyl-3-methyl-pentyl)-p-phenylendiamin
N,N'-Bis(1-methyl-heptyl)-p-phenylendiamin
N,N'-Diphenyl-p-phenylendiamin
N,N'-Di-(naphtyl-2-)-p-phenylendiamin
N-Isopropyl-N'-phenyl-p-phenylendiamin
N-(1,3-Dimethyl-butyl)-N'-phenyl-p-phenylendiamin
N-(1-Methyl-heptyl)-N'-phenyl-p-phenylendiamin
N-Cyclohexyl-N'-phenyl-p-phenylendiamin
4-(p-Toluol-sulfonamido)-diphenylamin
N,N'-Dimethyl-N,N'-di-sec.-butyl-p-phenylendiamin
Diphenylamin
4-Isopropoxy-diphenylamin
N-Phenyl-1-naphthylamin
N-Phenyl-2-naphthylamin
octyliertes Diphenylamin
4-Butylamino-phenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert.-butyl-4-dimethylamino-methyl-phenol
2,4'-Diamino-diphenylmethan
4,4'-Diamino-diphenylmethan
N,N,N,N'-Tetramethyl-4,4'-diamino-phenylmethan
1,2-Di-[(2-methyl-phenyl)-amino]-ethan
1,2-Di-(phenylamino)-propan
(o-Tolyl)-biguanid
Di-[4-(1',3'-dimethyl-butyl)-phenyl]amin
tert-octyliertes N-Phenyl-1-naphthylamin
Gemisch aus mono- und dialkylierten tert-Butyl-/tert-Octyldiphenylaminen.

**Beispiele für Metallpassivatoren sind:**

für Kupfer, z. B.
Benztriazol, Tetrahydro-benztriazol, 2-Mercaptobenzthiazol, 2,5-Dimercaptothiadiazol, Salicyliden-propylendiamin, Salze von Salicylaminoguanidin.

**Beispiele für Rost-Inhibitoren sind:**

a) Organinische Säuren, ihre Ester, Metallsalze und Anhydride, z. B. N-Oleyl-sarcosin, Sorbitan-mono-oleat. Blei-naphthenat, Dodecenylbernsteinsäure-anhydrid, Alkenylbernsteinsäure-Halbester, 4-Nonylphenoxy-essigsäure.

b) Stickstoffhaltige Verbindungen, z. B.:
I. Primäre, sekundäre oder tertiäre aliphatische oder cycloaliphatische Amine und Amin-Salze von organischen und anorganischen Säuren z. B. öllösliche Alkylammoniumcarboxylate.
II. Heterocyclische Verbindungen z. B.:
Substituierte Imidazoline und Oxazoline.
c) Phosphorhaltige Verbindungen, z. B.:
Aminsalze von Phosphorsäurepartialestern.
d) Schwefelhaltige Verbindungen, z. B.:
Barium-dinonylnaphthalin-sulfonate, Calciumpetroleum-sulfonate.

**Beispiele für Viscositätsindex-Verbesserer sind z. B.**

Polymethacrylate, Vinylpyrrolidon/Methacrylat-Copolymere, Polybutene, Olefin-Copolymere, Styrol/Acrylat-Copolymere.

**Beispiele für Stockpunkterniedriger sind z. B.:**

Polymethacrylat, alkylierte Naphthalinderivate.

**Beispiele für Dispergiermittel/Tenside sind z. B.:**

Polybutenylbernsteinsäure-imide, Polybutenylphosphonsäurederivate, basische Magnesium-, Calcium-, und Bariumsulfonate und -phenolate.

**Beispiele für Hochdruck- und/oder verschleissmindernde Additive sind z. B.:**

Schwefel und/oder Phosphor und/oder Halogen enthaltende Verbindungen, wie geschwefelte pflanzliche Öle, Zinkdialkyldithio-phosphate, Tritolyl-phospat, chlorierte Paraffine, Alkyl- und Aryldisulfide.

Beispiele für Antioxidantien und die anderen zusätzlichen Additive in Systemen auf Wasser-Basis sind die gleichen, wie sie für Systeme auf Öl-Basis aufgeführt sind, sofern sie wasserlöslich sind.

Beispiele für Puffersubstanzen sind Borax und Triethanolamin; Beispiele für Biocide sind 2,4,5-Trichlorphenol sowie die Na-Salze von 2,2'-Dihydroxy-5,5'-dichlordiphenylmethan und Orthophenylphenol und Beispiele von Schaumbrechern und Silicone.

Die neuen Verbindungen der Formel I weisen sowohl ausgezeichnete Eigenschaften als Metalldesaktivatoren wie gute Löslichkeit in funktionelle Flüssigkeiten sowohl auf Öl- wie auch Wasser-Basis auf. Zudem bilden sie keine unlöslichen Salze, wenn sie zusammen mit Zinkhaltigen Antiverschleiss-Additiven wie z. B. Zink-Dialkyldithiophosphaten eingesetzt werden.

In den folgenden Beispielen beziehen sich alle Angaben bezüglich Anteile und Prozente auf Gewichtsanteile resp. -Prozente.

**Beispiel 1:**

3.5 Teile Triazol und 12.05 Teile Di-2-ethyl-hexylamin werden mit 100 Teilen Methanol gemischt, worauf 4.05 Teile 36-%-iges wässriges Formalin zugegeben werden. Die Mischung wird 3 Stunden zum Rückfluss erhitzt. Anschliessend wird das Lösungsmittel bei vermindertem Druck (Wasserstrahlvakuum) entfernt. Auf diese Weise wird 1-Di-2-ethylhexylaminomethyltriazol in 100 % Ausbeute v. d. Th. erhalten. Das Produkt hat einen Sdp. von 189°C bei 0.07 mbar.

**Beispiel 2:**

7 Teile Triazol, 12.9 Teile Di-n-butylamin und 8.1 Teile 37-%-iges wässriges Formalin werden mit 100 Teilen Methanol gemischt und analog zu Beispiel 1 umgesetzt, was 20.4 Teile (97 % d.Th.) 1-Di-n-butylaminomethyltriazol mit einem Sdp. 105°C bei 0.075 mbar ergibt.

**Beispiel 3:**

13.8 Teile Triazol, 26.8 Teile Oleylamin und 16.2 Teile 37-%-iges wässriges Formalin werden mit 100 Teilen Methanol gemischt und analog zu Beispiel 1 umgesetzt, was 41.7 Teile N,N-Bis-(1-triazolylmethyl)-oleylamin als viskoses Öl ergibt.

**Beispiel 4:**

10.5 Teile Diethanolamin werden zu einer klaren Lösung von 6.9 Teilen Triazol und 8.1 Teilen 36-%-igem Formalin in 100 Teilen Wasser gegeben. Die Mischung wird 8 Stunden zum Rückfluss erhitzt, worauf das Lösungsmittel bei vermindertem Druck entfernt wird. Es wird so 1-[2,2'-Dihydroxyethyl)amino-methyl]triazol mit einem Sdp. von 115°C bei 0.08 mbar in 95 % d.Th. erhalten.

**Beispiel 5:**

Eine 0.05-%-ige Lösung des Produkts aus Beispiel 1 in Mineralöl der Turbinenölqualität, das zusätzlich 50 ppm gelösten Schwefel enthält, wird hergestellt.

Ein Kupferstreifen (60 x 10 x 1 mm) wird mit Siliciumcarbidpulver der Körnung 100, das mit einem Petrolether genetzten Wattebausch aufgenommen wird, poliert. Der polierte Streifen wird sogleich vollständig in die vorbereitete Lösung eingetaucht, welche 2 Stunden auf 100° C erhitzt wird. Nach dieser Zeit wird der Streifen herausgenommen, mit Petrolether gewaschen, getrocknet und seine Farbe mit der Kupferstreifen Korrosions Standardtafel gemäss ASTM D 130 verglichen. Bei dieser Bewertung entspricht 1 keiner bis leichter Anlauffarbe, 2 mässiger Anlauffarbe, 3 dunkler Anlauffarbe und 4 entspricht Korrosion; A, B, D bedeuten Abstufungen in der numerischen Einteilung.

Die Resultate sind in Tabelle 1 wiedergegeben.

**Tabelle 1**

| Beispiel | Aussehen des Metalls/Bewertung | Aussehen der Lösung |
|---|---|---|
| Kontrolle | Magenta 3B | Farblos |
| 5 | keine Veränderung 1A | Farblos |

**Beispiel 6:**

Eine 0.05-%-ige Lösung des Produkts aus Beispiel 4 in Wasser, das 0.132 g/l $MgSO_4 \cdot 7H_2O$ und 0.68 g/l $CaCl_2 \cdot 6H_2O$ enthält (Wasser gemäss DIN 51 360-Test), wird bergestellt und der pH-Wert der Lösung mit NaOH auf 7 - 8 eingestellt.

Ein Stück Kupferfolie (20 x 50 x 0.1 mm) wird durch Reiben mit Watte, welche mit Wasser und gepulvertem Bimsstein getränkt ist, gereinigt, getrocknet und gewogen. Danach wird sie vollständig in 50 ml der vorbereiteten Lösung in einer 60 ml Flasche mit Schraubverschluss eingetaucht. Die Flasche wird 24 Stunden in einen Ofen bei 70° C gebracht. Nach dieser Zeit wird der Streifen herausgenommen, gewaschen, getrocknet und die Farbe gemäss der ASTM D 130 Farbbewertung bestimmt. Anschliessend wird die Folie für 15 Sekunden in 5N Salzsäure bei 120° C getaucht, gewaschen, getrocknet und gewogen.

Die Resultate sind in Tabelle 2 wiedergegeben.

**Tabelle 2**

| Beispiel | Gewichtsverlust (mg) | Aussehen des Metalls Bewertung | Aussehen der Lösung |
|---|---|---|---|
| Kontrolle | 4.6 | Purpur-Schwarz Bewertung 4D | Farblos |
| 6 | 0.4 | Keine Veränderung Bewertung 1A | Farblos |

**Beispiel 7:**

6.9 Teile Triazol, 18.1 Teile Dicyclohexylamin und 8.1 Teile 37-%-iges wässriges Formalin werden mit 100 Teilen Methanol gemischt und analog zu Beispiel 1 umgesetzt. Es werden 24 Teile (92 % d.Th.) 1-N,N-Dicylcohexylaminomethyltriazol vom Smp. 62 - 64° C erhalten.

**Beispiel 8:**

Eine 0.05-%-ige Lösung des Produkts aus Beispiel 7 in Mineralöl der Turbinenqualität wird hergestellt und wie in Beispiel 5 geprüft.

Die Resultate sind in Tabelle 3 wiedergegeben.

**Tabelle 3**

| Beispiel | Aussehen des Metalls Bewertung | Aussehen der Lösung |
|---|---|---|
| Kontrolle | Magenta - Bewertung 3B | Farblos |
| 8 | Keine Veränderung Bewertung 1A | Farblos |

**Beispiel 9:**

6.9 Teile Triazol, 9.7 Teile Diallylamin und 8.1 Teile 37-%-iges wässriges Formalin werden mit 100 Teilen Methanol gemischt und analog zu Beispiel 1 umgesetzt. Es werden 16.4 Teile (89 % d.Th.) 1-N,N-Diallylamino-methyltriazol vom Sdp. 140°C bei 0.2 mbar erhalten.

**Beispiel 10:**

13.8 Teile Triazol, 9.3 Teile Anilin und 16.2 Teile 37-%-iges wässriges Formalin werden mit 100 Teilen Methanol gemischt und analog zu Beispiel 1 umgesetzt. Es werden 19.6 Teile (78 % d.Th.) N,N-Bis-(triazolylmethylen)-anilin als viskoses Öl erhalten.

**Beispiel 11:**

27.6 Teile Triazol, 6 Teile Diaminoethan und 32.4 Teile 37-%-iges wässriges Formalin werden mit 100 Teilen Methanol gemischt und analog zu Beispiel 1 umgesetzt. Es werden 37.3 Teile (97 % d.Th.) N,N,N',N'-Tetra-(triazolylmethylen)-diaminoethan vom Smp. 127 - 129°C erhalten.

**Beispiel 12:**

34.5 Teile Triazol, 10.3 Teile Diethylenamin und 40.5 Teile 37 %-iges wässriges Formalin werden mit 100 Teilen Methanol gemischt und analog zu Beispiel 1 umgesetzt. Es werden 44.2 Teile (95 % d.Th.) N,N,N',N",N"-Penta-(triazolylmethylen)-diethylentriazin als viskoses oranges Öl erhalten.

**Patentansprüche** für die Vertragsstaaten: BE, DE, FR, IT

1. Zusammensetzung enthaltend eine funktionelle Flüssigkeit und, als Metalldesaktivator, einen metalldesaktivierenden Anteil einer Verbindung der Formel I

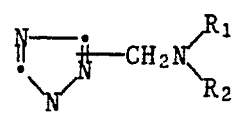

I

worin $R_1$ und $R_2$, gleich oder verschieden, $C_1$-$C_{20}$-Alkyl, $C_3$-$C_{20}$-Alkenyl, $C_5$-$C_{12}$-Cycloalkyl, $C_7$-$C_{13}$-Aralkyl, $C_6$- oder $C_{10}$-Aryl bedeuten, oder zusammen mit dem Stickstoffatom, an das sie gebunden sind, einen 5 - 7 gliedrigen heterozyklischen Rest bilden, oder worin $R_1$ und $R_2$ einen Rest der Formel II

$R_3X[(Alkylen)O]_n(Alkylen)-$ II

bedeuten, worin X O, S oder N, $R_3$ Wasserstoff oder $C_1$-$C_{20}$-Alkyl und Alkylen einen $C_1$-$C_{12}$-Alkylen-Rest, und n Null oder eine ganze Zahl von 1 bis 6, bedeuten, oder $R_1$ hat die vorstehend beschriebene Bedeutung und $R_2$ bedeutet einen Rest der Formel III

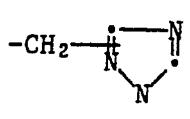

III

oder $R_2$ bedeutet einen Rest der Formel III wie vorstehend definiert und $R_1$ bedeutet einen Rest der Formel IV

-[Alkylen]$_n$-N($R_4$)-A-[N($R_4$)$_2$]$_m$ IV

worin m Null oder 1 ist und A im Fall von m = Null einen Rest der Formel III und im Fall von m = 1 Alkylen oder $C_6$-$C_{10}$-Arylen bedeutet und Alkylen und n die vorstehend definierten Bedeutungen haben und $R_4$ einen Rest der Formel III, wie vorstehend beschrieben, bedeutet.

2. Zusammensetzung gemäss Anspruch 1 worin der Anteil der Verbindung der Formel I 0.001 Gew.-% bis 5 Gew.-% bezogen auf das Gewicht der gesamten Zusammensetzung beträgt.

3. Neue N-substituierte Verbindungen der Formel I

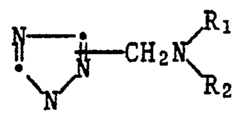

I

worin $R_1$ und $R_2$, gleich oder verschieden, $C_1$-$C_{20}$-Alkyl, $C_3$-$C_{20}$-Alkenyl, $C_5$-$C_{12}$-Cycloalkyl, $C_7$-$C_{13}$-Aralkyl, $C_6$- oder $C_{10}$-Aryl bedeuten, oder zusammen mit dem Stickstoffatom, an das sie gebunden sind, einen 5 - 7 gliedrigen heterozyklischen Rest bilden, oder worin $R_1$ und $R_2$ einen Rest der Formel II

$R_3$X[(Alkylen)O]$_n$(Alkylen)- II

bedeuten, worin X O, S oder N, $R_3$ Wasserstoff oder $C_1$-$C_{20}$-Alkyl und Alkylen einen $C_1$-$C_{12}$-Alkylen-Rest, und n Null oder eine ganze Zahl von 1 bis 6, bedeuten, oder $R_1$ hat die vorstehend beschriebene Bedeutung und $R_2$ bedeutet einen Rest der Formel III

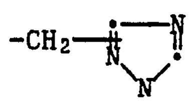

III

oder $R_2$ bedeutet einen Rest der Formel III wie vorstehend definiert und $R_1$ bedeutet einen Rest der Formel IV

-[Alkylen]$_n$-N($R_4$)-A-[N($R_4$)$_2$]$_m$ IV

worin m Null oder 1 ist und A im Fall von m = Null einen Rest der Formel III und im Fall von m = 1 Alkylen oder $C_6$-$C_{10}$-Arylen bedeutet und Alkylen und n die vorstehend definierten Bedeutungen haben und $R_4$ einen Rest der Formel III, wie vorstehend beschrieben, bedeutet, mit der Massgabe, dass die Verbindungen
1-Dimethylaminomethyl-triazol,
1-Diethylaminomethyl-triazol,
1-Di-n-octylaminomethyl-triazol,
1-Di(2-ethylhexyl)aminomethyl-triazol,
1-N-Methyl-N-octadecylaminomethyl-triazol,
1-Di-prop-2-enylaminomethyl-triazol,
1-N-Phenyl-N-benzylaminomethyl-triazol,
1-N-Methyl-N-cyclohexylaminomethyltriazol,
1-N-Methyl-N-phenylaminomethyl-triazol,
1-Dicyclohexylaminomethyl-triazol,
1-(-4-morpholinomethyl)-triazol,
1-(-4-piperidinomethyl)-triazol,
1-(-4-perhydroazepinomethyl)-triazol,
N,N-bis-(1-triazolylmethyl)-laurylamin,
N,N-bis-(1-triazolylmethyl)-cyclohexylamin,
N,N-bis-(1-triazolylmethyl)-phenylamin,
N,N-bis-(1-triazolylmethyl)-n-octylamin und
N,N-bis-(1-triazolylmethyl)-(2-ethylhexyl)amin
ausgenommen sind.

4. Verbindungen gemäss Anspruch 3, worin m = 1 ist.

5. Verfahren zur Herstellung von Verbindungen der Formel I, dadurch gekennzeichnet, dass man eine Verbindung der Formel V

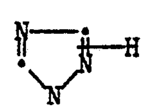

V

mit Formaldehyd und einem Amin der Formel VI

$HNR_1R_2$ VI

worin $R_1$ und $R_2$ die vorstehend beschriebene Bedeutung haben, umsetzt.

**Patentansprüche** für den Vertragsstaat: NL

1. Zusammensetzung enthaltend eine funktionelle Flüssigkeit und, als Metalldesaktivator, einen metalldesaktivierenden Anteil einer Verbindung der Formel I

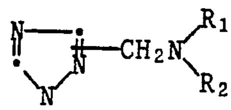 I

worin $R_1$ und $R_2$, gleich oder verschieden, $C_1$-$C_{20}$-Alkyl, $C_3$-$C_{20}$-Alkenyl, $C_5$-$C_{12}$-Cycloalkyl, $C_7$-$C_{13}$-Aralkyl, $C_6$- oder $C_{10}$-Aryl bedeuten, oder zusammen mit dem Stickstoffatom, an das sie gebunden sind, einen 5 - 7 gliedrigen heterozyklischen Rest bilden, oder worin $R_1$ und $R_2$ einen Rest der Formel II

$R_3X[(Alkylen)O]_n(Alkylen)$- II

bedeuten, worin X O, S oder N, $R_3$ Wasserstoff oder $C_1$-$C_{20}$-Alkyl und Alkylen einen $C_1$-$C_{12}$-Alkylen-Rest, und n Null oder eine ganze Zahl von 1 bis 6, bedeuten, oder $R_1$ hat die vorstehend beschriebene Bedeutung und $R_2$ bedeutet einen Rest der Formel III

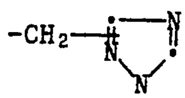 III

oder $R_2$ bedeutet einen Rest der Formel III wie vorstehend definiert und $R_1$ bedeutet einen Rest der Formel IV

$-[Alkylen]_n-N(R_4)-A-[N(R_4)_2]_m$ IV

worin m Null oder 1 ist und A im Fall von m = Null einen Rest der Formel III und im Fall von m = 1 Alkylen oder $C_6$-$C_{10}$-Arylen bedeutet und Alkylen und n die vorstehend definierten Bedeutungen haben und $R_4$ einen Rest der Formel III, wie vorstehend beschrieben, bedeutet.

2. Zusammensetzung gemäss Anspruch 1 worin der Anteil der Verbindung der Formel I 0.001 Gew.-% bis 5 Gew.-% bezogen auf das Gewicht der gesamten Zusammensetzung beträgt.

3. Neue N-substituierte Verbindungen der Formel I

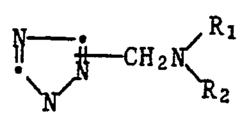 I

worin $R_1$ und $R_2$, gleich oder verschieden, $C_1$-$C_{20}$-Alkyl, $C_3$-$C_{20}$-Alkenyl, $C_5$-$C_{12}$-Cycloalkyl, $C_7$-$C_{13}$-Aralkyl, $C_6$- oder $C_{10}$-Aryl bedeuten, oder zusammen mit dem Stickstoffatom, an das sie gebunden sind, einen 5 - 7 gliedrigen heterozyklischen Rest bilden, oder worin $R_1$ und $R_2$ einen Rest der Formel II

$R_3X[(Alkylen)O]_n(Alkylen)$- II

bedeuten, worin X O, S oder N, $R_3$ Wasserstoff oder $C_1$-$C_{20}$-Alkyl und Alkylen einen $C_1$-$C_{12}$-Alkylen-Rest, und n Null oder eine ganze Zahl von 1 bis 6, bedeuten, oder $R_1$ hat die vorstehend beschriebene Bedeutung und $R_2$ bedeutet einen Rest der Formel III

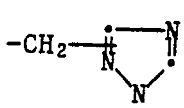 III

oder $R_2$ bedeutet einen Rest der Formel III wie vorstehend definiert und $R_1$ bedeutet einen Rest der Formel IV

$-[Alkylen]_n-N(R_4)-A-[N(R_4)_2]_m$ IV

worin m Null oder 1 ist und A im Fall von m = Null einen Rest der Formel III und im Fall von m = 1 Alkylen oder $C_6$-$C_{10}$-Arylen bedeutet und Alkylen und n die vorstehend definierten Bedeutungen haben und $R_4$ einen Rest der Formel III, wie vorstehend beschrieben, bedeutet, mit der Massgabe, dass die Verbindungen

1-N-Phenyl-N-Benzylaminomethyl-triazol,
1-Di-phenylaminomethyl-triazol,
1-(-4-Morpholinomethyl)-triazol und
1-(-4-Piperidinomethyl)-triazol
ausgenommen sind.

4. Verbindungen gemäss Anspruch 3, worin m = 1 ist.

5. Verfahren zur Herstellung von Verbindungen der Formel I, dadurch gekennzeichnet, dass man eine Verbindung der Formel V

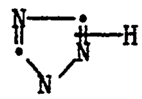 V

mit Formaldehyd und einem Amin der Formel VI

$HNR_1R_2$ VI

worin $R_1$ und $R_2$ die vorstehend beschriebene Bedeutung haben,umsetzt.

**Claims** for the Contracting States: BE, DE, FR, IT

1. A composition comprising a functional liquid and, as metal deactivator, a metal deactivating amount of a compound of formula I

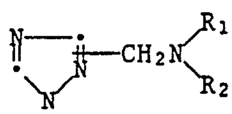 I

wherein $R_1$ and $R_2$ are the same or different and each is $C_1$-$C_{20}$alkyl, $C_3$-$C_{20}$alkenyl, $C_5$-$C_{12}$cycloalkyl, $C_7$-$C_{13}$aralkyl, $C_6$-$C_{10}$aryl, or $R_1$ and $R_2$, together with the nitrogen atom to which they are each attached, form a 5- to 7-membered heterocyclic radical, or $R_1$ and $R_2$ is each a radical of formula II

$R_3X[(alkylene)O]_n(alkylene)$- II

in which X is O, S or N, $R_3$ is hydrogen or $C_1$-$C_{20}$alkyl, "alkylene" is a $C_1$-$C_{12}$alkylene radical and n is 0 or an integer from 1 to 6, or $R_1$ has its previous significance and $R_2$ is a radical of formula III

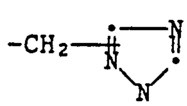 III

or $R_2$ is a radical of formula III as defined above and $R_1$ is a radical of formula IV:

$-[alkylene]_n-N(R_4)-A-[N(R_4)_2]_m$ IV

wherein m is zero or 1 and, when m is zero, A is a radical of formula III and, when m is 1, A is alkylene or $C_6$-$C_{10}$arylene, and alkylene and n are as previously defined and $R_4$ is a radical of formula III as hereinbefore defined.

2. A composition according to claim 1, wherein the amount of compound of formula I is 0.001 % by weight to 5 % by weight, based on the weight of the total composition.

3. A novel N-substituted compound of formula I

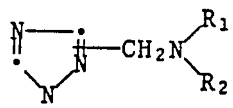

I

wherein $R_1$ and $R_2$ are the same or different and each is $C_1$-$C_{20}$alkyl, $C_3$-$C_{20}$alkenyl, $C_5$-$C_{12}$cycloalkyl, $C_7$-$C_{13}$aralkyl, $C_6$-$C_{10}$aryl, or $R_1$ and $R_2$, together with the nitrogen atom to which they are each attached, form a 5- to 7-membered heterocyclic radical, or $R_1$ and $R_2$ is each a radical of formula II

$R_3X[(alkylene)O]_n(alkylene)$- II

in which X is O, S or N, $R_3$ is hydrogen or $C_1$-$C_{20}$alkyl, "alkylene" is a $C_1$-$C_{12}$alkylene radical and n is 0 or an integer from 1 to 6, or $R_1$ has its previous significance and $R_2$ is a radical of formula III

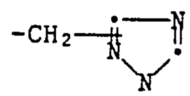

III

or $R_2$ is a radical of formula III as defined above and $R_1$ is a radical of formula IV:

$-[alkylene]_n-N(R_4)-A-[N(R_4)_2]_m$ IV

wherein m is zero or 1 and, when m is zero, A is a radical of formula III and, when m is 1, A is alkylene or $C_6$-$C_{10}$arylene, and alkylene and n are as previously defined and $R_4$ is a radical of formula III as hereinbefore defined, with the proviso that the compounds
1-dimethylaminomethyltriazole,
1-diethylaminomethyltriazole,
1-bis(n-octylaminomethyl)triazole,
1-bis(2-ethylhexyl)aminomethyltriazole,
1-N-methyl-N-octadecylaminomethyltriazole,
1-bis(prop-2-enylaminomethyl)triazole,
1-N-phenyl-N-benzylaminomethyltriazole,
1-N-methyl-N-cyclohexylaminomethyltriazole,
1-N-methyl-N-phenylaminomethyltriazole,
1-dicyclohexylaminomethyltriazole,
1-(4-morpholinomethyl)triazole,
1-(4-piperidinomethyl)triazole,
1-(4-perhydroazepinomethyl)triazole,
N,N-bis(1-triazolylmethyl)laurylamine,
N,N-bis(1-triazolylmethyl)cyclohexylamine,
N,N-bis(1-triazolylmethyl)phenylamine,
N,N-bis(1-triazolylmethyl)-n-octylamine and
N,N-bis(1-triazolylmethyl)-(2-ethylhexyl)amine
are excluded.

4. A compound according to claim 3, wherein m is 1.

5. A process for the preparation of a compound of formula I, which comprises reacting a compound of formula V

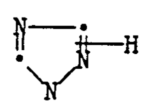

V

with formaldehyde and an amine of formula VI

$HNR_1R_2$ VI

wherein $R_1$ and $R_2$ are as previously defined.

**Claims** for the Contracting State: NL

1. A composition comprising a functional liquid and, as metal deactivator, a metal deactivating amount of a compound of formula I

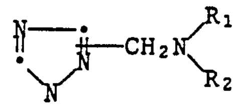

I

wherein $R_1$ and $R_2$ are the same or different and each is $C_1$-$C_{20}$alkyl, $C_3$-$C_{20}$alkenyl, $C_5$-$C_{12}$cycloalkyl, $C_7$-$C_{13}$aralkyl, $C_6$-$C_{10}$aryl, or $R_1$ and $R_2$, together with the nitrogen atom to which they are each attached, form a 5- to 7-membered heterocyclic radical, or $R_1$ and $R_2$ is each a radical of formula II

$R_3X[(alkylene)O]_n(alkylene)$- II

in which X is O, S or N, $R_3$ is hydrogen or $C_1$-$C_{20}$alkyl, "alkylene" is a $C_1$-$C_{12}$alkylene radical and n is 0 or an integer from 1 to 6, or $R_1$ has its previous significance and $R_2$ is a radical of formula III

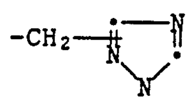

III

or $R_2$ is a radical of formula III as defined above and $R_1$ is a radical of formula IV:

$-[alkylene]_n-N(R_4)-A-[N(R_4)_2]_m$ IV

wherein m is zero or 1 and, when m is zero, A is a radical of formula III and, when m is 1, A is alkylene or $C_6$-$C_{10}$arylene, and alkylene and n are as previously defined and $R_4$ is a radical of formula III as hereinbefore defined.

2. A composition according to claim 1, wherein the amount of compound of formula I is 0.001 % by weight to 5 % by weight, based on the weight of the total composition.

3. A novel N-substituted compound of formula I

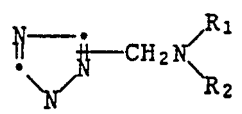

I

wherein $R_1$ and $R_2$ are the same or different and each is $C_1$-$C_{20}$alkyl, $C_3$-$C_{20}$alkenyl, $C_5$-$C_{12}$cycloalkyl, $C_7$-$C_{13}$aralkyl, $C_6$-$C_{10}$aryl, or $R_1$ and $R_2$, together with the nitrogen atom to which they are each attached, form a 5- to 7-membered heterocyclic radical, or $R_1$ and $R_2$ is each a radical of formula II

$R_3X[(alkylene)O]_n$-(alkylene)- II

in which X is O, S or N, $R_3$ is hydrogen or $C_1$-$C_{20}$alkyl, "alkylene" is a $C_1$-$C_{12}$alkylene radical and n is 0 or an integer from 1 to 6, or $R_1$ has its previous significance and $R_2$ is a radical of formula III

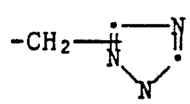

III

or $R_2$ is a radical of formula III as defined above and $R_1$ is a radical of formula IV:

$-[alkylene]_n-N(R_4)-A-[N(R_4)_2]$ IV

wherein m is zero or 1 and, when m is zero, A is a radical of formula III and, when m is 1, A is alkylene or $C_6$-

$C_{10}$arylene, and alkylene and n are as previously defined and $R_4$ is a radical of formula III as hereinbefore defined, with the proviso that the compounds

1-N-phenyl-N-benzylaminomethyltriazole,
1-bis(phenylaminomethyl)triazole,
1-(4-morpholinomethyl)triazole and
1-(4-piperidinomethyl)triazole
are excluded.

4. A compound according to claim 3, wherein m is 1.

5. A process for the preparation of a compound of formula I, which comprises reacting a compound of formula V

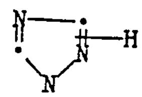

V

with formaldehyde and an amine of formula VI

$$HNR_1R_2 \qquad VI$$

wherein $R_1$ and $R_2$ are as previously defined.

**Revendications** pour les Etats contractants: BE, DE, FR, IT

1. Composition contenant un liquide fonctionnel et, comme désactivant de métaux, une proportion efficace, pour ce qui est de la désactivation des métaux, d'un composé répondant à la formule I:

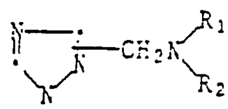

(I)

dans laquelle

$R_1$ et $R_2$ représentent chacun, indépendamment l'un de l'autre, un alkyle en $C_1$-$C_{20}$, un alcényle en $C_3$-$C_{20}$, un cycloalkyle en $C_5$-$C_{12}$, un aralkyle en $C_7$-$C_{13}$ ou un aryle en $C_6$ ou $C_{10}$, ou forment ensemble, et avec l'atome d'azote auquel ils sont liés, un radical hétérocyclique contenant de 5 à 7 maillons, ou

$R_1$ et $R_2$ représentent chacun un radical répondant à la formule II:

$$R_3X[(alkylène)O]n(alkylène)- \qquad (II)$$

dans laquelle X représente O, S ou N, $R_3$ représente l'hydrogène ou un alkyle en $C_1$-$C_{20}$, "alkylène" représente un radical alkylène en $C_1$-$C_{12}$, et n désigne un nombre entier de 0 à 6, ou

$R_1$ a la signification qui lui a été donnée ci-dessus et $R_2$ représente un radical de formule III:

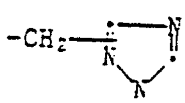

(III),

ou $R_2$ représente un radical de formule III tel que défini ci-dessus et $R_1$ représente un radical répondant à la formule IV :

$$-[alkylène]_n-N(R_4)-A-[N(R_4)_2]_m \qquad (IV)$$

dans laquelle m est égal à 0 ou à 1, A représente, dans le cas où m est nul, un radical de formule III et, dans le cas où m est égal à 1, un alkylène ou un arylène en $C_6$ ou $C_{10}$, "alkylène" et n ont les significations qui leur ont été données ci-dessus, et $R_4$ représente un radical de formule III tel que défini plus haut.

2. Composition selon la revendication 1 dans laquelle la proportion du composé de formule I est comprise entre 0,001 et 5 % en poids par rapport au poids de l'ensemble de la composition.

3. Nouveaux composés N-substitués répondant à la formule I:

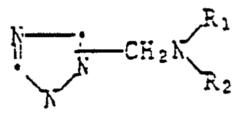 (I)

dans laquelle

$R_1$ et $R_2$ représentent chacun indépendamment l'un de l'autre, un alkyle en $C_1$-$C_{20}$, un alcényle en $C_3$-$C_{20}$, un cycloalkyle en $C_5$-$C_{12}$, un aralkyle en $C_7$-$C_{13}$ ou un aryle en $C_6$ ou $C_{10}$, ou forment ensemble, et avec l'atome d'azote auquel ils sont liés, un radical hétérocyclique contenant de 5 à 7 maillons, ou

$R_1$ et $R_2$ représentent chacun un radical répondant à la formule II:

$R_3X$[(alkylène)O]$_n$(alkylène)- (II)

dans laquelle X représente O, S ou N, $R_3$ représente l'hydrogène ou un alkyle en $C_1$-$C_{20}$, "alkylène" représente un radical alkylène en $C_1$-$C_{12}$, et n désigne un nombre entier de 0 à 6, ou

$R_1$ a la signification qui lui a été donnée ci-dessus et $R_2$ représente un radical de formule III:

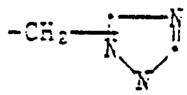 (III),

ou $R_2$ représente un radical de formule III tel que défini ci-dessus et $R_1$ représente un radical répondant à la formule IV:

-[alkylène]$_n$-N($R_4$)-A-[N($R_4$)$_2$]$_m$ (IV)

dans laquelle m est égal à 0 ou à 1, A représente, dans le cas où m est nul, un radical de formule III et, dans le cas où m est égal à 1, un alkylène ou un arylène en $C_6$ ou $C_{10}$, "alkylène" et n ont les significations qui leur ont été données ci-dessus, et $R_4$ représente un radical de formule III tel que défini ci-dessus,

à l'exception des composés suivants :
le diméthylaminométhyl-1 triazole,
le diéthylaminométhyl-1 triazole,
le di-n-octylaminométhyl-1 triazole,
le [bis-(éthyl-2 hexyl)-aminométhyl]-1 triazole,
le (N-méthyl-N-octadécylaminométhyl)-1 triazole,
le [di-(propène-2-yl)-aminométhyl]-1 triazole,
le (N-phényl-N-benzylaminométhyl)-1 triazole,
le (N-méthyl-N-cyclohexylaminométhyl)-1 triazole,
le (N-méthyl-N-phénylaminométhyl)-1 triazole,
le dicyclohexylaminométhyl-1 triazole,
le morpholinométhyl-1 triazole,
le pipéridinométhyl-1 triazole,
le (perhydroazépinyl-1 méthyl)-1 triazole,
la N,N-bis-(triazolyl-1 méthyl)-laurylamine,
la N,N-bis-(triazolyl-1 méthyl)-cyclohexylamine,
la N,N-bis-(triazolyl-1 méthyl)-phénylamine,
la N,N-bis-(triazolyl-1 méthyl)-n-octylamine et
la N,N-bis-(triazolyl-1 méthyl)-(éthyl-2 hexyl)-amine.

4. Composés selon la revendication 3 dans lesquels m est égal 1.

5. Procédé de préparation de composés de formule I, procédé caractérisé en ce qu'on fait réagir un composé de formule V :

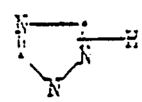 (V)

avec le formaldéhyde et une amine répondant à la formule VI:

$HNR_1R_2$ (VI)

dans laquelle $R_1$ et $R_2$ ont les significations qui leur ont données ci-dessus.

**Revendications** pour l'Etat contractant: NL

1. Composition contenant un liquide fonctionnel et, comme désactivant de métaux, une proportion efficace, pour ce qui est de la désactivation des métaux, d'un composé répondant à la formule I:

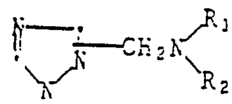

(I)

dans laquelle

$R_1$ et $R_2$ représentent chacun, indépendamment l'un de l'autre, un alkyle en $C_1$-$C_{20}$, un alcényle en $C_3$-$C_{20}$, un cycloalkyle en $C_5$-$C_{12}$, un aralkyle en $C_7$-$C_{13}$ ou un aryle en $C_6$ ou $C_{10}$, ou forment ensemble, et avec l'atome d'azote auquel ils sont liés, un radical hétérocyclique contenant de 5 à 7 maillons, ou

$R_1$ et $R_2$ représentent chacun un radical répondant à la formule II:

$R_3X[(\text{alkylène})O]_n(\text{alkylène})$- (II)

dans laquelle X représente O, S ou N, $R_3$ représente l'hydrogène ou un alkyle en $C_1$-$C_{20}$, "alkylène" représente un radical alkylène en $C_1$-$C_{12}$, et n désigne un nombre entier de 0 à 6, ou

$R_1$ a la signification qui lui a été donnée ci-dessus et $R_2$ représente un radical de formule III:

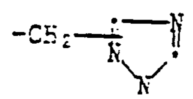

(III),

ou $R_2$ représente un radical de formule III tel que défini ci-dessus et $R_1$ représente un radical répondant à la formule IV:

$-[\text{alkylène}]_n-N(R_4)-A-[N(R_4)_2]_m$ (IV)

dans laquelle m est égal à 0 ou à 1, A représente, dans le cas où m est nul, un radical de formule III et, dans le cas où m est égal à 1, un alkylène ou un arylène en $C_6$ ou $C_{10}$, "alkylène" et n ont les significations qui leur ont été données ci-dessus, et $R_4$ représente un radical de formule III tel que défini plus haut.

2. Composition selon la revendication 1 dans laquelle la proportion du composé de formule I est comprise entre 0,001 et 5 % en poids par rapport au poids de l'ensemble de la composition.

3. Nouveaux composés N-substitués répondant à la formule I:

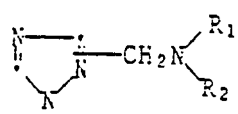

(I)

dans laquelle

$R_1$ et $R_2$ représentent chacun, indépendamment l'un de l'autre, un alkyle en $C_1$-$C_{20}$, un alcényle en $C_3$-$C_{20}$, un cycloalkyle en $C_5$-$C_{12}$, un aralkyle en $C_7$-$C_{13}$ ou un aryle en $C_6$ ou $C_{10}$, ou forment ensemble, et avec l'atome d'azote auquel ils sont liés, un radical hétérocyclique contenant de 5 à 7 maillons, ou

$R_1$ et $R_2$ représentent chacun un radical répondant à la formule II:

$R_3X[(\text{alkylène})O]_n(\text{alkylène})$- (II)

dans laquelle X représente O, S ou N, $R_3$ représente l'hydrogène ou un alkyle en $C_1$-$C_{20}$, "alkylène" représente un radical alkylène en $C_1$-$C_{12}$, et n désigne un nombre entier de 0 à 6, ou

$R_1$ a la signification qui lui a été donnée ci-dessus et $R_2$ représente un radical de formule III:

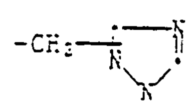

(III),

ou $R_2$ représente un radical de formule III tel que défini ci-dessus et $R_1$ représente un radical répondant à la formule IV:

$-[\text{alkylène}]_n-N(R_4)-A-[N(R_4)_2]_m$ (IV)

dans laquelle m est égal à 0 ou à 1, A représente, dans le cas où m est nul, un radical de formule III et, dans le cas où m est égal à 1, un alkylène ou un arylène en $C_6$ ou $C_{10}$, "alkylène" et n ont les significations qui leur ont été données ci-dessus, et $R_4$ représente un radical de formule III tel que défini ci-dessus, l'exception des composés suivants :

le (N-phényl-N-benzylaminométhyl)-1 triazole,
le diphénylaminométhyl-1 triazole,
le morpholinométhyl-1 triazole et
le (pipéridyl-1 méthyl)-1 triazole.

4. Composés selon la revendication 3 dans lesquels m est égal 1.

5. Procédé de préparation de composés de formule I, procédé caractérisé en ce qu'on fait réagir un composé de formule V:

5 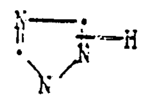 (V)

avec le formaldéhyde et une amine répondant à la formule VI:

$HNR_1R_2$ (VI)

dans laquelle $R_1$ et $R_2$ ont les significations qui leur ont données ci-dessus.